# EUROPÄISCHE PATENTSCHRIFT

(11) **EP 0 970 698 B1**
(45) Veröffentlichungstag und Bekanntmachung des Hinweises auf die Patenterteilung: **06.10.2004**
(21) Anmeldenummer: 99112790.3
(22) Anmeldetag: 02.07.1999
(51) Int. Cl.: A61K 31/7004, A61K 31/7016, A61K 31/702, A61K 31/7024, A61P 43/00

(54) **Verwendung von D-Galaktose zur Verhinderung von Nekrosen**
Use of D-galactose for preventing necrosis
Utilisation de D-galactose pour prévenir la necrose

(30) Priorität: 03.07.1998 DE 19829844
(43) Veröffentlichungstag der Anmeldung: 12.01.2000
(73) Patentinhaber: Afting, Ernst-Günter, Prof. Dr. Dr., 82152 Krailling (DE)
(72) Erfinder: Afting, Ernst-Günter, Prof. Dr. Dr., 82152 Krailling (DE)
(74) Vertreter: Bösl, Raphael, Dr. rer. nat., Dipl.-Chem.

(56) Entgegenhaltungen:
- EP-A- 0 922 459
- DE-A- 3 935 906
- DATABASE WPI Section Ch, Week 199251 Derwent Publications Ltd., London, GB; Class B04, AN 1992-418587 XP002118886 & JP 04 312533 A (NIPPON KAYAKU KK), 4. November 1992 (1992-11-04)
- DATABASE WPI Section Ch, Week 199308 Derwent Publications Ltd., London, GB; Class B05, AN 1993-061598 XP002118887 & JP 05 009114 A (TAKEDA CHEM IND LTD), 19. Januar 1993 (1993-01-19)

## Beschreibung

Die vorliegende Erfindung betrifft die Verwendung von D-Galaktose und/oder einem D-Galaktose-Derivat ausgewählt aus D-Galaktose-7-phosphat, UDP-D-Galaktose, Disaccharide der D-Galaktose, Trisaccharide der D-Galaktose und/oder Galaktoseester zur Verhinderung bzw. Verringerung von Nekrosen oder Zell- bzw. Gewebsschädigungen wobei die Nekrosen oder Zell- bzw. Gewebsschädigungen durch einen Infarkt oder durch eine diabetische Nekropathie verursacht werden.

Jede Form von Leben ist von Energie abhängig. Energieträger für den zellulären Stoffwechsel sind vor allem Kohlenhydrate, Fette und Proteine. Bei Mikroorganismen sind diese Nährsubstrate zur Aufrechterhaltung des Energie- und Baustoffwechsels neben anderen lebenswichtigen Substanzen wie Ionen, Schwermetallen, Vitaminen in der Umwelt bzw. bei der Fermentation in der Nährlösung zu finden. In höheren, komplexen Organismen wie den Wirbeltieren und dem Menschen geschieht der Antransport dieser Nährsubstrate über das Blut- und Gefäßsystem.

Höhere Organismen sind daher immer auf eine intakte Blutversorgung der Zellen angewiesen. Wird die Blutversorgung über einen gewissen Zeitraum hinaus unterbrochen, sistiert die Nährstoffversorgung der Gewebe, beginnt die Phase des Zelltodes (Nekrose oder Apoptose) und die Zellen sterben letztendlich ab. Die bei einem Zelltod und anschließender Zellyse freiwerdenden toxischen Substanzen können zudem das umliegende Gewebe über einen relativ weiten Bereich schädigen. Bei einer Unterbrechung der Blutversorgung ist es daher notwendig, die Überlebensfähigkeit der Zellen im unterversorgten Zellbereich sicherzustellen.

Je nach Art der Zellen, Gewebe und Organe werden die Nährsubstrate zum Energie- und Baustoffwechsel in unterschiedlicher Weise umgesetzt. Beispielsweise gibt es im Muskelgewebe höherer Lebewesen unterschiedliche Arten von Muskelfasern mit unterschiedlicher Stoffwechselleistung. Zum einen finden sich die nur Glucose-verbrennenden anaerob arbeitenden Muskelfasern, die verschiedene Hexosen wie auch polymere Zucker, z.B. Glykogen, in Glucose umsetzen und die Glucose immer, auch bei optimaler Sauerstoffversorgung, anaerob in Milchsäure umsetzen. Dabei werden nur zwei ATP pro Mol Glucose gewonnen. Der Vorteil dieser Muskelfasern ist die schnelle Kontraktionskraft und der Nachteil die geringe Dauerleistung. Zum anderen gibt es die aerob arbeitenden Muskelfasern, die Glucose und Fettsäuren verwerten und diese Substrate in der Atmungskette zu Kohlendioxid verbrennen. Sie sind daher immer auf eine optimale Sauerstoffversorgung angewiesen. Bei diesem aeroben Prozeß der Verbrennung von Glucose zu Kohlendioxid entstehen in der Glykolyse und anschließenden Atmungskette 36 Mol ATP pro Mol Glucose und somit ein Vielfaches der anaerob in der Glykolyse erhaltbaren Energie.

Zu den aerob arbeitenden Muskeln gehört auch der Herzmuskel. Alle aerob arbeitenden Muskeln können bei Unterbrechung der Blutzufuhr und damit der Nähr- und Sauerstoffversorgung die Glykolyse nutzen, d.h. die Glucose nur bis zur Milchsäure und nicht weiter zu Kohlendioxid umsetzen. Wegen der geringen Energiebilanz der Glykolyse und dem hohen Energieverbrauch des arbeitenden Muskels, wie z.B. des Herzens, reicht die Energiereserve bei mangelnder oder fehlender Blutversorgung sowie physiologischer Plasma- und Gewebekonzentrationen von Hexosen und Fettsäuren jedoch nur kurzfristig aus. Die Zellen sterben daher nach einer gewissen Latenzzeit ab.

Der Glucose-Spiegel im Blut wird durch den Körper auf einen Wert von etwa 5 mM eingestellt. Wird dieser Blutspiegel durch Nahrungszufuhr überoder durch Verbrauch unterschritten, so läuft ein komplexer hormoneller Regelkreis an, der den normalen Blutspiegel von 5 mM Glucose schnell wiederhergestellt.

Bei einer Unterbrechung der Blutversorgung in einem Muskel und dem damit drohenden Abbruch des Energie- und Baustoffwechsels der Zellen kann im allgemeinen Glucose in die Randbezirke der Mangelversorgung passiv hineindiffundieren. Ein normaler Blut- und Gewebespiegel von 5 mM Glucose reicht jedoch im allgemeinen wegen der geringen Größe des Glucose-Gradienten und der relativ langen Diffusionszeiten nicht aus, um die Nährstoffversorgung der Zellen ausreichend zu gewährleisten. Dieser Mangel kann auch nicht durch eine Erhöhung des Glucose-Spiegels im Blut ausgeglichen werden, da aufgrund der hormonellen Kontrolle die Glucose-Konzentration in kürzester Zeit wieder auf den Normalwert von 5 mM durch den Körper eingestellt wird. Zudem ist auch der Transport von Glucose aus dem Blut in die Muskelzelle Insulin-abhängig.

Da Insulin nicht von den Muskelzellen selbst, sondern für den gesamten Körper nur von den Langerhans'schen Zellen des Pankreas synthetisiert und von diesen zur Regulation des Glucose-Spiegels ins Blut ausgeschüttet wird, wird bei einer Mangelversorgung des Gewebes nicht nur die Nährstoff- und Sauerstoff-Versorgung unterbrochen, sondern auch die Insulinversorgung. Dies hat zur Folge, daß bei einer Unterbrechung des Blutkreislaufs, beispielsweise durch einen Thrombus, neben den Nährsubstraten und Sauerstoff auch kein Insulin an das postthrombische Muskelgewebe herankommt und die Glucose-Aufnahme in die Muskelzelle auch bei noch vorhandenen minimalen Mengen an Glucose nicht erfolgen kann.

Derartige pathologische Mechanismen laufen im allgemeinen bei allen Gefäßverschlüssen im Muskel ab, so vor allem beim Herzinfarkt, da Umgehungskreisläufe im Herzen nicht existieren. Die Größe der Mangelversorgung bestimmt die Größe des Infarktes und die Größe des Infarktbezirkes bei einem Herzinfarkt das klinische Bild des Patienten und seine Überlebenschance. Je kleiner der Infarktbezirk bzw. je mehr dieser durch eine schnelle medizinische Intervention begrenzt werden kann, desto geringer sind die bleibenden Schäden am Herzen und desto höher ist die Rehabilitationswahrscheinlichkeit des Patienten.

Die beschriebenen pathologischen Mechanismen laufen auch bei peripheren Verschlüssen in den Extremitäten ab, wo allerdings unter Umständen ein Umgehungskreislauf eine geringe Blutversorgung aufrecht erhalten kann und der Muskel nur bei Beanspruchung energetisch unterversorgt wird, was zum sogenannten Bewegungsschmerz führt.

Auch beim diabetischen Formenkreis wird durch die fortschreitende Mikroangiopathie im Gewebe die Blutversorgung zunehmend schlechter und Energie- und Stoffwechselsubstrate können die Zelle nicht mehr in ausreichendem Maße erreichen. Gangrän und Amputation sind daher im allgemeinen die langfristigen Folgen einer diabetischen Mikropathie.

Bei einem Gehirninfarkt läuft ein ähnlicher pathologischer Mechanismus ab, da die Nervenzellen des Gehirns nur Glucose als Nährsubstrat und keine anderen Nährsubstrate wie Fettsäuren oder Aminosäuren verwenden können. Die Glucose wird im Gehirn über die Glykolyse bis zum Pyruvat und dieses anschließend über die Atmungskette bis zum Kohlendioxid verbrannt. Auch im Gehirn bestehen wie im Herzen keine Umgehungskreisläufe, die beispielsweise bei einem Thrombus in einem Gefäß über ein anderes Gefäß die Substratversorgung sicherstellen können. Hinzu kommt, daß Nervenzellen unter normalen, physiologischen Umständen nicht regenerieren, so daß ein einmaliger Schaden schwere und im allgemeinen nicht behebbare Folgen hat. Daher ist eine schnelle Wiederherstellung der Substratversorgung, vor allem mit Glucose, von großer Bedeutung. Jedoch führt eine Erhöhung des Glucose-Spiegels im Blut zu keiner wesentlichen Verbesserung, da aufgrund der bereits geschilderten hormonellen Gegenregulation eine ausreichend hohe Glucose-Konzentration um den Infarktbereich herum nicht gebildet werden kann.

Aufgabe der vorliegenden Erfindung war es daher, durch Mangeldurchblutung verursachte Nekrosen oder Zell- bzw. Gewebsschädigungen zu verhindern bzw. zu verringern.

Es wurde nun überraschenderweise gefunden, daß die orale und/oder parenterale Verabreichung relativ hoher Konzentrationen an D-Galaktose die oben näher beschriebenen pathologischen Mechanismen verringert bzw. unterbricht und somit die nekrotischen Prozesse oder Zell- bzw. Gewebsschädigungen verhindern bzw. zumindest verringern kann.

Gegenstand der vorliegenden Erfindung ist daher die Verwendung von D-Galaktose und/oder einem D-Galaktose-Derivat ausgewählt aus D-Galaktose-7-phosphat, UDP-D-Galaktose, Disaccharide der D-Galaktose, Trisaccharide der D-Galaktose und/oder Galaktoseester zur Verhinderung bzw. Verringerung von Nekrosen oder Zell- bzw. Gewebsschädigungen, die insbesondere durch eine Mangeldurchblutung der Zellen bzw. Gewebe verursacht werden, wobei die Nekrosen oder Zell- bzw. Gewebsschädigungen durch einen Infarkt, insbesondere durch einen Muskelinfarkt und/oder Gehirninfarkt, vor allem durch einen Herzinfarkt, oder durch eine diabetische Nekropathie verursacht werden.

Freie D-Galaktose kommt in der Nahrung normalerweise nur in geringen Konzentrationen und in der Milch als Disaccharid Lactose vor. In der Zelle ist D-Galaktose in freier Form nicht vorhanden. Wird nun D-Galaktose und/oder ein Derivat davon z.B. bei einem Muskelinfarkt, wie einem Herzinfarkt, in relativ hoher Konzentration gegeben, kann D-Galaktose aufgrund der relativ hohen Konzentration in Blut und Gewebe relativ schnell in die Zellen des nekrotischen Bezirks diffundieren. Die schnelle Diffusion in die Zellen wird auch dadurch begünstigt, daß der Transport von D-Galactose über die Zellmembran in allen Organen und Geweben im Gegensatz zur Glucose nicht insulinabhängig ist.

In der Zelle wird die aufgenommene D-Galaktose durch die Galaktose-1-Kinase in Galaktose-1-Phosphat umgewandelt, das über UDP-Galaktose in UDP-Glucose umgewandelt und in Form von Glucose-1-Phosphat in den Energiestoffwechsel eingeschleust werden kann. Das Gleichgewicht zwischen UDP-Galaktose und UDP-Glucose liegt bei 1 : 3,5 und somit ganz auf der Seite der UDP-Glucose, so daß D-Glucose immer in ausreichendem Maße für den Energiestoffwechsel zur Verfügung steht. Die Konzentration von UDP-Galaktose ist jedoch im allgemeinen immer noch ausreichend, um den Baustoffwechsel ausreichend zu versorgen. Durch eine verhältnismäßig schnelle Gabe von relativ hohen D-Galaktose-Mengen und/oder deren Derivate kann daher ein relativ hoher Gewebespiegel von D-Galaktose um einen Infarktbezirk aufgebaut werden, so daß D-Galaktose schnell in den Infarktbezirk hineindiffundieren, die Zellen mit Energie- und Bausubstraten versorgen und somit die ansonsten ablaufenden Zell- bzw. Gewebsschädigungen unterbrechen kann. Aufgrund der relativ ungünstigen Energiebilanz beim anaeroben Stoffwechsel (Glykolyse) im mangelversorgten Gewebe ist daher ein hoher D-Galaktose-Durchsatz zu Glukose-1-Phosphat bevorzugt, so daß die Diffusion in das Infarktgewebe von einem relativ hohen D-Galaktose-Gradienten um das Infarktgewebe herum ausgehen kann.

Auch bei einem Gehirninfarkt wird gemäß der vorliegenden Erfindung je nach Pathogenese im allgemeinen eine Besserung bzw. Eingrenzung der Penumbra durch relativ hohe D-Galaktose-Gaben und/oder deren Derivate erreicht. Analog wie beim Muskel kann überraschenderweise durch eine relativ schnelle orale und/oder vorzugsweise parenterale Verabreichung von D-Galaktose und/oder deren Derivate eine vorteilhafte Wirkung auf das Überleben der Nervenzellen erreicht werden.

Die physiologischen Mechanismen sind ähnlich wie die Muskelgewebe. Die D-Galaktose diffundiert aus dem beispielsweise durch eine Infusion aufgebauten hohen D-Galaktose-Spiegel, um den Infarkt in die Penumbra hinein und wird in der Nervenzelle hormonunabhängig aufgenommen. Die D-Galaktose wird anschließend in der Nervenzelle zu D-Galaktose-1-Phosphat phosphoryliert und über UDP-Galaktose und UDP-Glucose zu D-Glucose-1-Phosphat umgewandelt, das anschließend in die Glykolyse eingespeist wird. D-Glucose-1-Phosphat kann jedoch im allgemeinen wegen der unterbrochenen Sauerstoffversorgung nicht bis Kohlendioxid umgesetzt werden. Aufgrund der ablaufenden anaeroben Glykolyse wird jedoch ausreichend Energie bereitgestellt, um einen Minimalstoffwechsel in der Zelle zum Erhalt der Zelle sicherzustellen.

Die in der Zelle ebenso vorhandene UDP-Galaktose ist zudem ein wichtiges Ausgangsprodukt für den Baustoffwechsel, der folglich auch nicht mehr blockiert ist und vor allem den Zellmembranen die notwendige Stabilität verleihen kann. Folglich kann eine relativ schnelle D-Galaktose-Verabreichung auch beim Gehirninfarkt nekrotische Schäden eindämmen oder verhindern bzw. irreparable Schäden einschränken oder verhindern.

Anstelle von oder zusätzlich zur D-Galaktose kann gemäß der vorliegenden Erfindung auch ein oder mehrere D-Galaktose-Derivate, ausgewählt aus D-Galaktose-1-Phosphat, UDP-D-Galaktose (UDP-Galaktose), Di- und Trisaccharide der D-Galaktose sowie Galaktoseester, einer Verbindungsform, die durch eine physiologische Spaltung im Körper zu monomerer D-Galaktose führt, eingesetzt werden.

Im allgemeinen wird D-Galaktose bzw. deren Derivat in einer Menge von ca. 1-25 g, vorzugsweise ca. 2-18 g, insbesondere ca. 3-12 g, vor allem ca. 3-10 g pro oraler Applikationsform verwendet. Geeignete Applikationsformen sind beispielsweise eine Tablette oder ein Pulver, insbesondere eine Brausetablette, da hierdurch eine Selbstmedikation besonders einfach möglich ist. Die Tablette oder das Pulver kann auch noch weitere Zusatzstoffe enthalten, z.B. Bicarbonat im Falle einer Brausetablette. Vorzugsweise wird die Tablette oder das Pulver auf nüchternen Magen verabreicht.

D-Galaktose bzw. deren Derivat kann auch in einer Konzentration von 5-230 g pro 250 ml, vorzugsweise 25-180 g pro 250 ml, insbesondere 50-100 g pro 250 ml, vor allem 70 g pro 250 ml entsprechend 0,01 - 4 g pro kg Körpergewicht verwendet werden. Hierbei ist es besonders vorteilhaft, wenn die D-Galaktose bzw. deren Derivat in Form einer Infusionslösung vorliegt, da diese entweder direkt oder über einen Bypass als Zusatz zu einer bereits existierenden Infusionslösung dem Patienten intravenös gegeben werden kann. Hierdurch können insbesondere größere Mengen z.B. als Bolus verabreicht werden, um die vorteilhaften erfindungsgemäßen Wirkungen besonders schnell erreichen zu können. Eine weitere vorteilhafte Applikationsform ist ein Sirup enthaltend D-Galaktose bzw. deren Derivat. Zur Zubereitung der Lösung bzw. des Sirups kann man beispielsweise von einer 50%-igen D-Galaktose-Lösung ausgehen.

Gemäß der vorliegenden Erfindung können auch noch zusammen mit D-Galaktose bzw. deren Derivat weitere Zusatzstoffe verwendet werden. Besonders vorteilhaft als Zusatzstoffe sind α-Ketoglutarsäure und/oder Ornithin, da diese Zusatzstoffe z.B. giftige Ammoniumionen abfangen, welche in untoxische Aminocarbonsäuren überführt und in der untoxischen Form im Harnstoff-Zyklus als Harnstoff gebunden werden, der dann ausgeschieden wird. Als Salze dieser Zusatzstoffe eignen sich insbesondere das Natrium-, Kalium-, Magnesium-, Zink- und/oder Calciumsalz. Das Calciumsalz hat zusätzlich den Vorteil, daß gleichzeitig die Gefäße abgedichtet werden. Das Zinksalz hat den Vorteil, daß es ein potentielles Defizit der Zink-abhängigen Enzyme des Ammonium-entgiftenden Harnstoff-Zyklus ausgleicht.

α-Ketoglutarsäure oder ein Salz davon liegt beispielsweise in einer Menge von 50-300 mg, vorzugsweise 75-250 mg, insbesondere 100-200 mg, vor allem 150 mg pro Applikationsform vor und Ornithin oder ein Salz davon in einer Menge von 50-300 mg, vorzugsweise 75-250 mg, insbesondere 100-200 mg, vor allem 150 mg pro Applikationsform. Im Fall einer Lösung liegt α-Ketoglutarsäure oder ein Salz davon beispielsweise in einer Konzentration von 5-100 mM, vorzugsweise 10-75 mM, insbesondere 20-50 mM, vor allem 25 mM vor, und Ornithin oder ein Salz davon beispielsweise in einer Konzentration von 1-20 mM, vorzugsweise 2-15 mM, insbesondere 3-10 mM, vor allem 5 mM.

Weitere geeignete Zusätze sind beispielsweise Monosaccharide, Vitamine, anorganische Elektrolyte, insbesondere ein Magnesium- und/oder Zinksalz in metabolisch notwendigen Dosen, beispielsweise in Form eines der oben genannten Salze, Aromastoffe und Zusätze zur pH-Regulierung, Pufferung, Stabilisierung und Konservierung, wobei beispielsweise 5-100 Gew.-%, vorzugsweise mindesiens 10-100 Gew.-%, insbesondere 20-100 Gew.-%, vor allem ca. 20 Gew.-% des Monosaccharidanteils, eine Verbindung ausgewählt aus D-Mannose, D-Glucosamin, D-Lactose und/oder D-Lactulose oder Gemische davon ist, wobei mindestens 40 Mol-%, vorzugsweise mindestens 50 Mol-%, insbesondere mindestens 75 Mol-% dieser Verbindungen auf D-Galaktose entfallen soll. Im Falle der Disaccharide Lactose oder Lactulose werden diese wie Monosaccharide gerechnet. Dasselbe gilt für Invert- oder Rohrzuckerpräparate in bezug auf D-Glucose oder D-Fructose. Bei Vorhandensein von z.B. D-Galaktose und D-Mannose ist das Verhältnis von D-Galaktose zu D-Mannose im allgemeinen 200 : 1 bis 2 : 1. Ein anderer Zusatzstoff, insbesondere bei einer Infusionslösung, kann γ-Globulin oder Albumin als Beispiel eines supplementierenden Proteins, vorzugsweise in einer Menge von 0,05-1 Gew.-%, bezogen auf 100 g Kohlenhydrate verwendet werden.

Als Vitamin eignet sich insbesondere Vitamin C, welches im Falle einer flüssigen Zusammensetzung in einer Konzentration von 1-20 mM, vorzugsweise 2-15 mM, insbesondere 3-10 mM, vor allem 5 mM vorliegt und im Falle einer festen Zusammensetzung in einer Menge von 50-300 mg, vorzugsweise 75-250 mg, insbesondere 100-200 mg, vor allem 150 mg.

Besonders bevorzugt ist es, wenn neben D-Galaktose und/oder ein D-Galaktose-Derivat auch α-Ketoglutarsäure oder ein Salz davon, Ornithin oder ein Salz davon und Vitamin C verwendet wird. Durch die Zusätze α-Ketoglutarsäure und Ornithin werden die Giftstoffe im Körper besonders schnell abgefangen. Vitamin C wirkt als Reduktionsäquivalent, das insbesondere in metabolischen Streßsituationen in verminderter Form vorliegt. Neben Ornithin kann die Zusammensetzung auch noch andere essentielle Aminosäuren, beispielsweise in einer Konzentration von 1-15 g pro Liter, vorzugsweise 2-9 g pro Liter je Aminosäure enthalten. Üblicherweise sind Ornithin und die zusätzlichen Aminosäuren in derselben Menge bzw. Konzentration vorhanden.

Als Elektrolyte sind beispielsweise Natriumchlorid, Kaliumchlorid, Calciumchlorid, Zinkchlorid und/oder Magnesiumchlorid geeignet, wobei das Verhältnis der einwertigen zu den zweiwertigen Kationen im allgemeinen im Bereich von 35 : 1 bis 10 : 1 liegt. Die Lösung enthält im allgemeinen insgesamt bis zu 200 mval/l Elektrolyte.

## Patentansprüche

1. Verwendung von D-Galaktose und/oder einem oder mehreren D-Galaktose-Derivat/en ausgewählt aus D-Galaktose-1-phosphat, UDP-D-Galaktose, Disaccharide der D-Galaktose, Trisaccharide der D-Galaktose und/oder Galaktoseester zur Verhinderung bzw. Verringerung von Nekrosen oder Zell- bzw. Gewebsschädigungen, **dadurch gekennzeichnet, daß** die Nekrosen oder Zell- bzw. Gewebsschädigungen durch einen Infarkt, insbesondere durch einen Muskelinfarkt und/oder Gehirninfarkt, vor allem durch einen Herzinfarkt, oder durch eine diabetische Nekropathie verursacht werden.

2. Verwendung nach Anspruch 1, **dadurch gekennzeichnet, daß** die Nekrosen oder Zell- bzw. Gewebsschädigungen durch Mangeldurchblutung verursacht werden.

3. Verwendung nach Anspruch 1 oder 2, **dadurch gekennzeichnet, daß** D-Galaktose und/oder ein oder mehrere D-Galaktose-Derivat/e in einer Menge von 1-25 g pro oraler Applikationsform verwendet wird.

4. Verwendung nach Anspruch 3, **dadurch gekennzeichnet, daß** D-Galaktose und/oder ein oder mehrere D-Galaktose-Derivat/e in Form einer Tablette oder eines Pulvers, insbesondere in Form einer Brausetablette verwendet wird.

5. Verwendung nach Anspruch 1 oder 2, **dadurch gekennzeichnet, daß** D-Galaktose und/oder ein oder mehrere D-Galaktose-Derivat/e in einer Konzentration von 5-230 g/250 ml verwendet wird.

6. Verwendung nach Anspruch 5, **dadurch gekennzeichnet, daß** D-Galaktose und/oder ein oder mehrere D-Galaktose-Derivat/e in Form einer Infusionslösung oder in Form eines Sirups verwendet wird.

7. Verwendung nach einem der Ansprüche 1-6, **dadurch gekennzeichnet, daß** D-Galaktose und/oder ein oder mehrere D-Galaktose-Derivate in Gegenwart von einem oder mehreren Zusatzstoffen verwendet wird.

8. Verwendung nach Anspruch 7, **dadurch gekennzeichnet, daß** α-Ketoglutarsäure oder ein Salz davon, Ornithin oder ein Salz davon, Monosaccharide, anorganische Elektrolyte, essentielle Aminosäuren und/oder Vitamin C als Zusatzstoffe verwendet werden.

9. Verwendung nach Anspruch 8, **dadurch gekennzeichnet, daß** als Salz das Natrium-, Kalium-, Magnesium-, Zink- und/oder das Calciumsalz, insbesondere das Zink- und/oder das Calciumsalz verwendet wird.

10. Verwendung nach Anspruch 8 oder 9, **dadurch gekennzeichnet, daß** α-Ketoglutarsäure, Ornithin und/oder Vitamin C in einer Menge von 50-300 mg pro Applikationsform verwendet wird.

## Claims

1. Use of D-galactose and/or one or more D-galactose derivative/s selected from D-galactose-1-phosphate, UDP-D-galactose, disaccharides of D-galactose, trisaccharides of D-galactose and/or galactose ester for the inhibition and reduction, respectively, of necroses or cell and tissue damages, respectively, **characterized in that** the necroses or cell and tissue damages, respectively, are caused by an infarction, particularly by a muscle infarction and/or brain infarction, primarily by a cardiac infarction, or by a diabetic necropathy.

2. Use according to claim 1, **characterized in that** the necroses or cell and tissue damages, respectively, are caused by insufficient blood circulation.

3. Use according to claim 1 or 2, **characterized in that** D-galactose and/or one or more D-galactose derivative/s are used in an amount of 1-25 g per oral application form.

4. Use according to claim 3, **characterized in that** D-galactose and/or one or more D-galactose derivative/s are used in the form of a tablet or a powder, particularly in the form of an effervescent tablet.

5. Use according to claim 1 or 2, **characterized in that** D-galactose and/or one or more D-galactose derivative/s are used in a concentration of 5-230 g/250 ml.

6. Use according to claim 5, **characterized in that** D-galactose and/or one or more D-galactose derivative/s are used in the form of an infusion solution or in the form of a sirup.

7. Use according to one of the claims 1-6, **characterized in that** D-galactose and/or one or more D-galactose derivatives are used in the presence of one or more additives.

8. Use according to claim 7, **characterized in that** α-ketoglutaric acid or a salt thereof, ornithine or a salt thereof, monosaccharides, anorganic electrolytes, essential amino acids and/or vitamine C are used as additives.

9. Use according to claim 8, **characterized in that** the sodium, potassium, magnesium, zinc and/or the calcium salt, particulary the zinc and/or the calcium salt are used as as salt.

10. Use according to claim 8 or 9, **characterized in that** α-ketoglutaric acid, ornithine and/or vitamine C are used in an amount of 50-300 mg per application form.

## Revendications

1. Utilisation de D-galactose et/ou d'un ou de plusieurs dérivé(s) de galactose choisi(s) parmi le D-galactose-1-phosphate, l'UDP-D-galactose, le disaccharide du D-galactose, le trisaccharide du D-galactose et/ou l'ester de galactose pour empêcher ou diminuer les nécroses ou les lésions cellulaires ou tissulaires, **caractérisée en ce que** les nécroses ou les lésions cellulaires ou tissulaires ont été provoquées par un infarctus, en particulier par un infarctus musculaire et/ou cérébral, essentiellement par un infarctus cardiaque ou par une nécropathie diabétique.

2. Utilisation selon la revendication 1, **caractérisée en ce que** les nécroses ou les lésions cellulaires ou tissulaires ont été provoquées par une irrigation déficiente.

3. Utilisation selon la revendication 1 ou 2, **caractérisée en ce que** le D-galactose et/ou un ou plusieurs dérivés de D-galactose sont utilisés dans une quantité d'environ 1 à 25 g par forme administrée oralement.

4. Utilisation selon la revendication 3, **caractérisée en ce que** le D-galactose et/ou un ou plusieurs dérivés de D-galactose sont utilisés sous forme de comprimé ou de poudre, en particulier sous forme de comprimé effervescent

5. Utilisation selon la revendication 1 ou 2, **caractérisée en ce que** le D-galactose et/ou un ou plusieurs dérivés de D-galactose sont utilisés à une concentration de 5 à 230 g/250 ml.

6. Utilisation selon la revendication 5, **caractérisée en ce que** le D-galactose et/ou un ou plusieurs dérivés de D-galactose sont utilisés sous forme de solution de perfusion ou sous forme de sirop.

7. Utilisation selon une des revendications 1 à 6, **caractérisée en ce que** le D-galactose et/ou un ou plusieurs dérivés de D-galactose sont utilisés en présence d'un ou de plusieurs additifs.

8. Utilisation selon la revendication 7, **caractérisée en ce que** l'on utilise comme additif l'acide α-cétoglutarique ou un de ses sels, l'ornithine ou un de ses sels, des monosaccharides, des électrolytes inorganiques, des acides aminés essentiels et/ou de la vitamine C.

9. Utilisation selon la revendication 8, **caractérisée en ce que** l'on utilise comme sel le sel de sodium, de potassium, de magnésium, de zinc et/ou de calcium, en particulier le sel de zinc et/ou de calcium.

10. Utilisation selon la revendication 8 ou 9, **caractérisée en ce que** l'acide α-cétoglutarique, l'ornithine ou la vitamine C sont utilisés dans une quantité d'environ 50 à 300 mg par forme administrée.
